(19) Europäisches Patentamt
European Patent Office
Office européen des brevets

(11) Publication number: **0 006 615**
**B1**

(12) **EUROPEAN PATENT SPECIFICATION**

(45) Date of publication of patent specification: **02.03.83**

(51) Int. Cl.³: **C 07 D 301/02, C 07 D 303/08**

(21) Application number: **79102138.9**

(22) Date of filing: **27.06.79**

(54) **Process for preparing epihalohydrin enantiomers.**

(30) Priority: **27.06.78 US 919590**
**08.09.78 US 940681**

(43) Date of publication of application:
**09.01.80 Bulletin 80/1**

(45) Publication of the grant of the patent:
**02.03.83 Bulletin 83/9**

(84) Designated Contracting States:
**AT BE CH DE FR GB IT LU NL SE**

(56) References cited:
**BEILSTEINS HANDBUCH DER ORGANISCHEN CHEMIE, 4th edition, 1st supplement, Vol. 17, 1934, SPRINGER VERLAG, Berlin**
**BEILSTEINS HANDBUCH DER ORGANISCHEN CHEMIE, 4th edition, 3rd and 4th supplement, Vol. 19, part 2 1977, SPRINGER-VERLAG, Berlin, Heidelberg, New York**
**Chemical Abstracts, Volume 65, Nr. 1, July 4, 1966 (COLUMBUS, OHIO, USA) N. NAKABAYSHI et al. "Some reactions of the glycidyl esters of sulfonic acids" column 671 D—H**

(73) Proprietor: **MERCK & CO. INC.**
**126, East Lincoln Avenue P.O. Box 2000**
**Rahway New Jersey 07065 (US)**

(72) Inventor: **Baldwin, John James**
**1680 Wagon Wheel Lane**
**Lansdale, Pennsylvania 19446 (US)**
Inventor: **McClure, David Earl**
**2307 Bramblegate Drive**
**Hatfield, Pennsylvania 19448 (US)**

(74) Representative: **Abitz, Walter, Dr.-Ing. et al,**
**Abitz, Morf, Gritschneder P.O. Box 86 01 09**
**D-8000 München 86 (DE)**

(56) References cited:
**Chemical Abstracts, Volume 87, Nr. 17 October 24, 1977 (COLUMBUS, OHIO, USA) B. T. GOLDING et al. "Rapid syntheses of 3-0-benzyl-sn-glycerol and 2-0-benzylglycerol" page 652, column 1, abstract Nr. 134 209E**

**EP 0 006 615 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing epihalohydrin enantiomers

*Background of the Invention*

The present invention is concerned with processes for preparing epihalohydrin enaı

Epibromohydrin enantiomers have been prepared by a resolution process—and an mers. hydrin enantiomer has been prepared from 1-bromo-3-chlorpropan-2-ol. enantiomer derived hloro- epibromohydrin [Chemische Berichte 48, 1862—1865, (1915)]. the

An improved method for preparing the epihalohydrin enantiomers from sulfonyloxyhaloaı has been discovered.

*Summary of the Invention:*

Process for preparing (S) or (R) epihalohydrin from an (S) or (R) sulfonyloxyhaloalcohol.

*Description of the Preferred Embodiments:*

An embodiment of the present invention is a process for preparing an enantiomer of epihalohydri which comprises treating an enantiomer of an alcohol having the formula

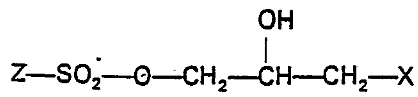

wherein Z is phenyl, monosubstituted phenyl, $CF_3$ or $C_1$—$C_6$ alkyl and X is Cl or Br with an alkali me glycolate and recovering said epihalohydrin by distillation.

The reaction is illustrated by the following equation:

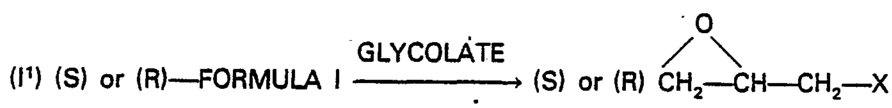


The product is recovered in good yield by direct vacuum distillation at room temperature from the reaction mixture.

Z may be $C_{1-6}$ alkyl, phenyl or phenyl monosubstituted by $C_{1-3}$ alkyl, p-$NO_2$, p-$OCH_3$ or 2-Cl. The phenyl group monosubstituted by $C_{1-3}$ alkyl is exemplified by p-propylphenyl, o-methylphenyl and m-ethylphenyl. The $C_{1-6}$ alkyl group includes $CH_3$, $C(CH_3)_3$, isopropyl, n-hexyl and the like. The p-methylphenyl and $CH_3$ groups are preferred.

The alkali metal glycolate includes K or Na ethylene glycolate, with Na ethylene glycolate being preferred.

When a more conventional low boiling solvent/strong base, e.g. methanol/$NaOCH_3$ is used in place of the glycolate in reaction (I1), the product yield obtained on direct vacuum distillation at room temperature is low.

Epihalohydrins have wide utility in organic synthesis. The enantiomers of epihalohydrin may be especially useful to prepare intermediates which in turn can be used to prepare specific isomers which have pharmaceutical utility. One such preparation is illustrated by the following reaction equations:

REACTION SEQUENCE A

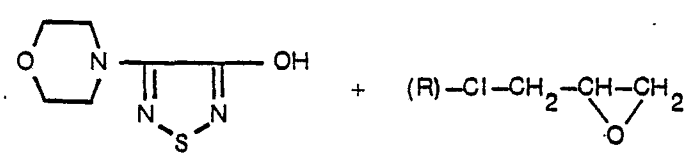

(A1) (B1)

STEP 1

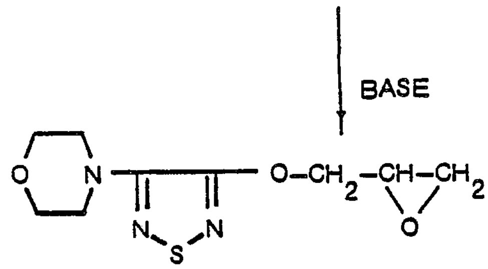


(S) –

(C)

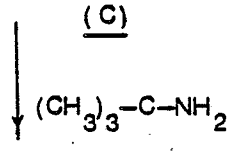


STEP 2

(S) –

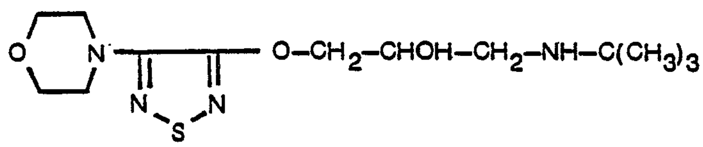

(D)

The product (*C*) is a commercial β-adrenergic blocking agent.

Other preparations utilizing the Step 1, sequence A type reaction are illustrated by the following reaction equation:

REACTION B

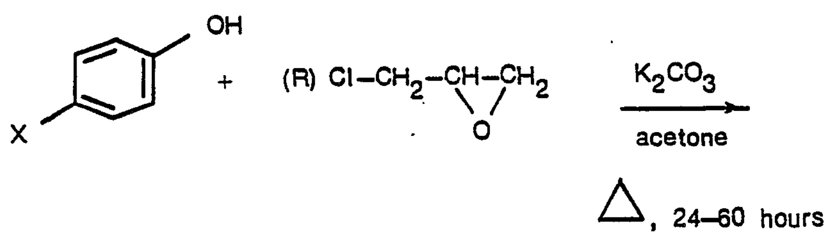


($A^{11}$) ($B^1$)

(S)

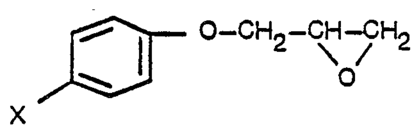

($C^1$)

X in this equation is H, CHO, CN or $OCH_3$.

Where a stronger base, such as NaH in DMF, is used in the above reactions, the intermediate (C or $C^1$) is obtained as a mixture of (S) and (R) isomer.

Steps (a) and (b) of the following example describe the preparation of starting and intermediate materials, whereas step (c) illustrates the process of the present invention. All temperatures are in °C.

(a) *(S)-Glycerol-1,2-acetonide*

To an ice-cooled solution of 1,2:5,6-di-O-isopropylidine-D-mannitol (80.0 g., 0.3 ml) in THF (400 m) was added portionwise with stirring dry $Pb(OAc)_4$ (134 g, 0.3 m), while maintaining the temperature below 10°C. The solution was stirred for 30 minutes with ice cooling and an additional 30 minutes without. After filtering through Super-Cel and cooling in an ice bath, a solution of $NaBH_4$ (22.9 g, 0.62 m) in 4% aqueous NaOH (400 ml) was added dropwise with vigorous stirring while maintaining the temperature below 10°C. After stirring in an ice bath for 30 minutes and at room temperature for 9 minutes, solid ammonium chloride was added to the solution until it buffered at about pH 8. The THF was removed under reduced pressure, and the resulting aqueous solution was saturated with NaCl. After extracting into ethyl acetate, the organic layer was washed with 5% aqueous NaOH saturated with NaCl, dried ($Na_2SO_4$), and concentrated. Distillation afforded pure (S)-glycerol-1,2-acetonide (58.4 g, 73%); bp 80—90°C/20 mm; $^1$H NHR ($CDCl_3$) 1.45 (6H, s), 3.5—4.5 (6H, m); $[^{25}_{D}$ = 11.3° (c = 5.174, $CH_3OH$).

(b) *(R)-3-Tosyloxy-1,2-propanediol*

To an ice-cooled solution of (S)-glycerol-1,2-acetonide (72.0 g 0.55 m) in pyridine (300 ml) was added portionwise with stirring p-toluenesulfonyl chloride (104.0 g, 0.55 m). After standing in a refrigerator for 16 hours, the reaction mixture was diluted with ether (300 ml), washed with 1*N*HCl until the aqueous wash was acidic, and then washed with saturated aqueous $NaHCO_3$. The ether layer was dried ($Na_2SO_4$) and concentrated to give (R)-3-tosyloxypropanediol acetonide (141.0 g, 91%), which was used without further purification.

The acetonide from above in acetone (100 ml) and 1*N*HCl (300 ml) was heated on a steam bath for 30 minutes. The resulting solution was concentrated to dryness, and the residue was dissolved in $CH_2Cl_2$. After drying ($Na_2SO_4$) and concentration, the resulting oil solidified upon standing. Residual solvents were removed at 25°C and 0.5 mm. over 18 hours to give (R)-3-tosyloxy-1,2-propanediol, (121.0 g, 100%); mp 54—59°C. (lit = 61—63°C.); $^1$H NMR ($CDCl_3$) 2.4 (3H, s), 3.3—4.3 (7H, m), 7.35 and 7.8 (4H, 2D, J = 8).

(c) *(S)-Epichlorohydrin*

To triphenylphosphine (13.2 g, 0.05 m) in $CCl_4$ (20 ml) and DMF (50 ml), (R)-3-tosyloxy-1,2-propanediol (12.3 g, 0.05 m) in DMF (50 ml) was added all at once. After the addition was complete, the temperature increased to 50°C. over 15 minutes. The mixture was then allowed to stir for 3 hours. The residual solvents were removed (50°C., 2 mm) and the residue was taken up in $H_2O$, dried ($Na_2SO_4$), and concentrated. Residual solvents were removed at 25°C and 0.2 mm over 18 hours.

To this residue, composed of triphenylphosphine oxide and (S)-3-tosyloxy-2-hydroxy-1-chloropropane, in dry ethylene glycol (50 ml) was added a solution of sodium ethylene glycolate [from sodium pellets (1.25 g, 0.054 m)] in dry ethylene glycol (50 ml). After stirring for 15 minutes, (S)-epichlorohydrin was distilled from the reaction mixture at room temperature and 0.2 mm and trapped in dry ice/acetone. The $^1$H NMR indicated that traces of $CH_2Cl_2$ and $H_2O$ were present; $[\alpha]^{20}_{D} = 28.1°$ (C = 2.47, $CH_3OH$).

A small sample was purified by preparative GC to yield pure (S)-epichlorohydrin $[\alpha]^{23}_{D} = 33.0°$ (c = 2.47, $CH_3OH$).

**Claims**

1. A process for preparing an enantiomer of epihalohydrin which comprises treating an enantiomer of an alcohol having the formula

$$Z{-}SO_2{-}O{-}CH_2{-}\overset{\displaystyle OH}{\overset{|}{C}H}{-}CH_2{-}X$$

wherein Z is $C_{1-6}$ alkyl, phenyl or phenyl monosubstituted by $C_{1-3}$ alkyl, p—$NO_2$, p—$OCH_3$ or 2—Cl and X is Cl or Br, with alkali metal glycolate and removing said epihalohydrin by destillation.

2. The process of claim 1 wherein Z is p-tolyl or methyl and the glycolate is sodium ethylene glycolate.

3. The process of claim 2 wherein said epihalohydrin and said alcohol are both the (S)-isomer and Z is p-tolyl.

4. The process of claim 2 wherein said epihalohydrin and said alcohol are both the (R)-isomer and z is methyl.

5. The process of claim 3 wherein said epihalohydrin is epichlorohydrin.

6. The process of claim 4 wherein said epihalohydrin is epichlorohydrin.

**Revendications**

1. Procédé de préparation d'un énantiomère d'épihalohydrine dans laquel on traite un énantiomère d'un alcool de formule:

$$Z-SO_2-O-CH_2-\overset{\displaystyle OH \atop |}{CH}-CH_2-X$$

où Z est un alcoyle en $C_1$ à $C_6$, un phényle ou un phényle monosubstitué par un alcoyle en $C_1$ à $C_3$, p-$NO_2$, p-$OCH_3$ ou 2-Cl et X est Cl ou Br, avec un glycolate de métal alcalin et on retire ladite épihalohydrine par distillation.

2. Procédé de la revendication 1 où Z est un p-tolyle ou un méthyle et le glycolate est l'éthylèneglycolate de sodium.

3. Procédé de la revendication 2 où ladite épihalohydrine et ledit alcool sont tous deux l'isomère (S) et Z est un p-tolyle.

4. Procédé de la revendication 2 où ladite épihalohydrine et ledit alcool sont tous deux l'isomère (R) et Z est un méthyle.

5. Procédé de la revendication 3 où ladite épihalohydrine est l'épichlorohydrine.

6. Procédé de la revendication 4 où ladite épihalohydrine est l'épichlorohydrine.

**Patentansprüche:**

1. Ein Verfahren zur Herstellung von Epihalohydrin-Enantiomeren, umfassend die Behandlung eines Enantiomers eines Alkohols der Formel

$$Z-SO_2-O-CH_2-\overset{\displaystyle OH \atop |}{CH}-CH_2-X$$

worin Z $C_{1-6}$-Alkyl, Phenyl oder durch $C_{1-3}$-Alkyl, p-$NO_2$, p-$OCH_3$ oder 2-Cl monosubstituiertes Phenyl ist und X Cl oder Br ist, mit Alkalimetallglycolat und das Entfernen des Epihalohydrins durch Destillation.

2. Das Verfahren nach Anspruch 1, worin Z p-Tolyl oder Methyl ist und das Glycolat Natriumethylenglycolat ist.

3. Das Verfahren nach Anspruch 2, worin sowohl das Epihalohydrin als auch der Alkohol (S)-Isomere sind und Z p-Tolyl ist.

4. Das Verfahren nach Anspruch 2, worin sowohl das Epihalohydrin als auch der Alkohol (R)-Isomere sind und Z Methyl ist.

5. Das Verfahren nach Anspruch 3, worin das Epihalohydrin Epichlorohydrin ist.

6. Das Verfahren nach Anspruch 4, worin das Epihalohydrin Epichlorohydrin ist.